# EUROPÄISCHE PATENTANMELDUNG

(11) **EP 2 740 537 A1**
(43) Veröffentlichungstag der Anmeldung: **11.06.2014**
(21) Anmeldenummer: 14158368.2
(22) Anmeldetag: 03.05.2013
(51) Int. Cl.: B01L 9/06, B65D 85/42, B65D 25/10

(54) **Haltestruktur zum gleichzeitigen Halten einer Mehrzahl von Behältern zur Aufbewahrung von Substanzen für medizinische, pharmazeutische oder kosmetische Anwendungen sowie Transport- oder Verpackungsbehälter mit Selbiger**

(30) Priorität: 03.05.2012 DE 102012103898; 03.05.2012 US 201261642154 P
(62) Teilanmeldung aus: 13166538.2
(71) Anmelder: Schott AG, 55122 Mainz (DE)
(72) Erfinder: Wissner, Kai, 69493 Hirschberg (DE); Pawlowski, Edgar, 55271 Stadecken-Elsheim (DE); Coulon, Joel, 9000 St. Gallen (CH); Dr.-Ing. Lovis, Ralph, 8400 Winterthur (CH); Heiter, Carmen, 9010 St. Gallen (CH)
(74) Vertreter: 2K Patentanwälte Blasberg Kewitz & Reichel

(57) **Zusammenfassung**

Offenbart wird eine Haltestruktur zum gleichzeitigen Halten einer Mehrzahl von Behältern (2) für Substanzen für medizinische, pharmazeutische oder kosmetische Anwendungen, wobei die Behälter (2) einen verengten Halsabschnitt (5) aufweisen, mit einem flächigen, rechteckförmigen Träger (25; 134), der eine Mehrzahl von Öffnungen oder Aufnahmen (32; 39; 87; 120) aufweist, die in einer regelmäßigen Anordnung angeordnet sind und die ausgelegt sind, sodass die Behälter in die zugeordneten Öffnungen oder Aufnahmen einführbar sind, und Mit Haltemitteln (145; 155), die einer jeweiligen Öffnung oder Aufnahme zugeordnet sind und ausgelegt sind, um die Mehrzahl von Behältern (2) an dem Träger (134) an vorbestimmten Positionen und in der regelmäßigen zweidimensionalen Anordnung zu halten.

Erfindungsgemäß sind die Haltemittel als Formschlusselemente (137; 140; 145; 155) ausgebildet sind, die als Klappen wirken, um die Behälter im Bereich ihrer verengten Halsabschnitte (5) an dem Träger formschlüssig zu fixieren. Weil die Klappen in ihrer ersten Stellung die zugeordnete Öffnung oder Aufnahme vollständig freigeben, können die Behälter so einfach und zuverlässig in die Öffnungen oder Aufnahmen des Träger eingeführt und wieder aus diesen entnommen werden.

## Beschreibung

Die vorliegende Anmeldung beansprucht die Priorität der Deutschen Patentanmeldung Nr. 10 2012 103 898.8 "Haltestruktur zum gleichzeitigen Halten einer Mehrzahl von medizinischen oder pharmazeutischen Behältern sowie Transport- oder Verpackungsbehälter mit Selbiger", angemeldet am 3. Mai 2012, deren gesamter Inhalt hiermit im Wege der Bezugnahme mit beinhaltet sei.

### Gebiet der Erfindung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen (Vials), und betrifft insbesondere die gleichzeitige Halterung einer Mehrzahl von Behältern in einer Haltestruktur in solcher Weise, dass diese, während diese in der gehalten werden, in Abfüll- oder Bearbeitungsanlagen weiter verarbeitet werden können, insbesondere in einem Steriltunnel, einer Abfüllanlage für flüssige medizinische oder pharmazeutische Anwendungen oder einem Gefriertrockenschrank hierfür.

### Hintergrund der Erfindung

Als Behälter zur Aufbewahrung von und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter (sog. Tubs) bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen (sog. Nests) eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die einzelnen Medikamentenbehälter werden zunächst lose in Aufnahmen, die in der Haltestruktur ausgebildet sind, angeordnet. Anschließend wird die Haltestruktur in den Transport- und Verpackungsbehälter eingesetzt und dieser mit einem gasundurchlässigen Kunststoffschlauch umgeben. Beim anschließenden Evakuieren der so ausgebildeten Verpackungseinheit wird der Kunststoffschlauch aufgrund des in dem Schlauch vorherrschenden Unterdrucks in die Zwischenräume zwischen den Medikamentenbehältern hineingedrückt, was so einerseits zu einer Stabilisierung der Position der Medikamentenbehälter in der Haltestruktur führt und andererseits eine weitere unkontrollierte Kollision von benachbarten Medikamentenbehältem verhindert. Beim Evakuieren und beim Anschließenden Öffnen des Kunststoffschlauchs können jedoch die Medikamentenbehälter seitlich verrutschen, was den Automatisierungsaufwand zur Weiterverarbeitung der Medikamentenbehälter erhöht. Ferner können die Medikamentenbehälter nach dem Öffnen des Kunststoffschlauchs dennoch unkontrolliert kollidieren, was die vorgenannten Nachteile mit sich bringt. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart. Zur Weiterverarbeitung müssen die Medikamentenbehälter jedoch stets vereinzelt werden. Eine chargenweise Weiterverarbeitung der Medikamentenbehälter, während diese in einer plattenförmigen Haltestruktur, wie vorstehend ausgeführt, aufgenommen sind, ist nicht möglich.

Jedenfalls ist ein unmittelbarer Kontakt der Böden der Medikamentenbehälter, insbesondere der Böden von Fläschchen, bei den herkömmlichen Haltestrukturen nicht möglich. Dies erschwert jedoch die Weiterverarbeitung der Medikamentenbehälter insbesondere dann, wenn deren Inhalt einer Gefriertrocknung (auch als Lyophilisation oder Sublimationstrocknung bezeichnet) unterzogen werden soll. Ferner ist eine Weiterverarbeitung der Medikamentenbehälter unmittelbar in den Haltestrukturen nicht möglich, da diese dort entweder starr gehalten werden oder für die Weiterverarbeitung nicht in ausreichendem Maß zugänglich sind, weshalb die Medikamentenbehälter für eine Weiteverarbeitung herkömmlich stets aus den Haltestrukturen entnommen werden müssen.

### Zusammenfassung der Erfindung

Aufgabe der vorliegenden Erfindung ist es, eine Haltestruktur zum gleichzeitigen Halten einer Mehrzahl von Behältern für medizinische oder pharmazeutische Anwendungen, insbesondere von Glasfläschchen, dahingehend weiterzubilden, dass die Behälter einfach und kostengünstig steril verpackt, wieder entpackt und weiterverarbeitet werden können. Gemäß einem bevorzugten weiteren Gesichtspunkt der vorliegenden Erfindung soll eine solche Haltestruktur insbesondere für eine Weiterverarbeitung der Behälter, während diese in der Haltestruktur gehalten werden, ausgelegt sein. Gemäß einem weiteren Gesichtspunkt der vorliegenden Erfindung soll ferner ein entsprechender Transport- und Verpackungsbehälter mit zumindest einer solchen Haltestruktur bereitgestellt werden.

Diese Aufgaben werden gemäß der vorliegenden Erfindung durch eine Haltestruktur mit den Merkmalen nach Anspruch 1 sowie durch einen Transport- und Verpackungsbehälter nach Anspruch 14 gelöst. Weitere vorteilhafte Ausführungsformen sind Gegenstand der rückbezogenen Unteransprüche.

Erfindungsgemäß sind die Behälter in der Haltestruktur formschlüssig gehalten. Zur formschlüssigen Halterung von zylindrischen Behältern steht eine Vielzahl von Haltemitteln zur Verfügung. Die gegenseitige Verschiebung zwischen Behälter und Haltestruktur ist verhindert, solange ein Verbindungspartner dem anderen Verbindungspartner im Wege steht, diesen also sperrt.

Gemäß einem ersten Gesichtspunkt wird eine Haltestruktur zum gleichzeitigen Halten einer Mehrzahl von Behältern für Substanzen für medizinische, pharmazeutische oder kosmetische Anwendungen bereitgestellt, mit einem flächigen, rechteckförmigen Träger, der eine Mehrzahl von Öffnungen, die in einer regelmäßigen Anordnung angeordnet sind, und diesen zugeordnete Haltemittel aufweist, um die Mehrzahl von Behältern an dem Träger formschlüssig zu halten, dadurch gekennzeichnet, dass die Haltemittel von einer Unterseite des Trägers abragen und ausgelegt sind, um die Behälter an deren oberen Rand formschlüssig zu halten, und dass auf einer der Unterseite gegenüber liegenden Oberseite des Trägers Aufnahmen vorgesehen sind, die ausgelegt sind, um Behälter auf der Oberseite des Trägers aufzunehmen.

Mit Hilfe der Haltemittel können die Behälter zuverlässig auf der Unterseite des Trägers gehalten werden. Gleichzeitig können mittels der Aufnahmen weitere Behälter auf der Oberseite des Trägers mit einer hohen Packungsdichte gehalten werden. Somit können erfindungsgemäß auch mehrere solche Haltestrukturen (sog. Nests) mit den daran gehaltenen Behältern in einer in einer gestapelten Anordnung übereinander gestapelt aufbewahrt werden, was erhebliche Vorteile bei der Verarbeitung und Handhabung der Behälter bietet, da Prozessierungsanlagen mit kleinerer Grundfläche eingesetzt werden können.

Die Haltemittel können dabei so ausgelegt sein, dass auch bereits verschlossene Behälter, beispielsweise mit einem Metalldeckel, der auf das obere Ende der Behälter oder Fläschchen aufgebördelt ist, an dem Träger gehalten werden können.

In den Aufnahmen können die Behälter dabei grundsätzlich beabstandet zu der Oberseite des Trägers aufgenommen sein. Gemäß einer weiteren Ausführungsform können die Aufnahmen auch so ausgelegt sein, dass Böden der Behälter unmittelbar auf der Oberseite des Trägers abgestützt sind.

Gemäß einer weiteren Ausführungsform können die Aufnahmen von umlaufenden Seitenwänden ausgebildet sein, deren Innendurchmesser bevorzugt auf den Außendurchmesser der darin aufzunehmenden Behälter abgestimmt ist. Beispielsweise können die unteren Enden der Behälter in den Aufnahmen geklemmt gehalten werden. Hierzu können die Aufnahmen insbesondere aus einem elastischen Kunststoff ausgebildet sein.

Gemäß einer weiteren Ausführungsform sind die Haltemittel als elastische Rasthaken ausgebildet, die von der Unterseite des Trägers abragen und um die Öffnungen des Trägers herum verteilt angeordnet sind. Bevorzugt sind die Rasthaken dabei so ausgelegt, um den oberen Rand der Behälter formschlüssig zu hintergreifen und diese so zu fixieren

Um die Behälter an dem Träger zu halten, werden die Rasthaken beim Einführen der Behälter elastisch auseinander gespreizt. Hierzu können die die Rasthaken eine untere Einführschräge aufweisen, die beim Einführen der Behälter von der Unterseite des Trägers her mit deren oberen Ende in Anlage gelangt und entlang der die oberen Enden der Behälter dann gleiten, um die Rasthaken auseinander zu spreizen.

Gemäß einer weiteren Ausführungsform weisen die Rasthaken weiterhin eine Schräge bzw. abgeschrägte Auflage- oder Anlagefläche auf, die der jeweiligen Öffnung des Trägers zugewandt ist und auf der der obere Rand des gehaltenen Behälters aufliegt. Soll der Behälter wieder von dem Träger abgenommen werden, so gleitet der obere Rand der Behälter beim Abziehen der Behälter nach unten entlang der abwärts geneigten Auflage- oder Anlagefläche, um die Rasthaken wieder elastisch auseinander zu spreizen. So wird das Abnehmen der Behälter von dem Träger erleichtert.

Gemäß einer bevorzugten Ausführungsform sind die Rasthaken einstückig mit dem Träger ausgebildet sind. Zweckmäßig sind auch die Aufnahmen auf der Oberseite des Träger einstückig mit diesem ausgebildet.

Gemäß einem ersten Gesichtspunkt wird eine Haltestruktur zum gleichzeitigen Halten einer Mehrzahl von Behältern für Substanzen für medizinische, pharmazeutische oder kosmetische Anwendungen bereitgestellt, mit einem flächigen, rechteckförmigen Träger, der eine Mehrzahl von Öffnungen oder Aufnahmen, die in einer regelmäßigen Anordnung angeordnet sind, und diesen zugeordnete Haltemittel aufweist, um die Mehrzahl von Behältern an dem Träger formschlüssig zu halten. Die Haltestruktur zeichnet sich erfindungsgemäß dadurch aus, dass die Haltemittel als Klappen ausgebildet sind, die um die Öffnungen oder Aufnahmen des Trägers herum verteilt angeordnet sind, wobei die einer jeweiligen Öffnung oder Aufnahme zugeordneten Klappen in einer ersten Stellung, in welcher die Öffnung oder Aufnahme vollständig freigegeben ist, weggeklappt sind und in einer zweiten Stellung in Richtung der Öffnung zurück geklappt sind, um gemeinsam einen oberen Rand des in der jeweiligen Öffnung oder Aufnahme aufgenommenen Behälters abstützen.

Weil die Klappen in ihrer ersten Stellung die zugeordnete Öffnung oder Aufnahme vollständig freigeben, können die Behälter so einfach und zuverlässig in die Öffnungen oder Aufnahmen des Träger eingeführt und wieder aus diesen entnommen werden.

Der Formschluss erfolgt zweckmäßig entweder unterhalb des aufgeweiteten oberen Randes der Behälter, d.h. im Bereich des verengten Hals- bzw. Nackenbereichs und unmittelbar unterhalb des oberen Rands, oder am unteren Ende der Behälter, beispielsweise an einem Boden der Behälter. Zweckmäßig ist der aufgeweitete obere Rand oder das untere Ende bzw. der Boden der Behälter auf den Formschlusselementen der Haltestruktur unmittelbar abgestützt. Alternativ kann der obere Rand oder das untere Ende bzw. der Boden der Behälter auch formschlüssig umgriffen oder hintergriffen werden. Einer bodenseitigen Abstützung der Behälter bedarf es erfindungsgemäß grundsätzlich nicht, sodass ein Zugriff auf die Unterseiten (Böden) der in der Haltestruktur gehaltenen Behälter grundsätzlich möglich ist. Dies ermöglicht erfindungsgemäß, dass die Behälter, während diese in der Haltestruktur aufgenommen sind, weiterverarbeitet werden können. Mit anderen Worten, die Behälter können in den Haltestrukturen chargenweise weiter verarbeitet werden, bleiben jedoch während der Weiterverarbeitung weiterhin zuverlässig und kollisionsfrei in den Haltestrukturen gehalten, was erhebliche Geschwindigkeitsvorteile und Vorteile bei der Automatisierung von Weiterverarbeitungsanlagen ermöglicht und so insgesamt zu noch wirtschaftlicheren und kostengünstigeren Prozessen führt. Die Behälter (Vials) können insbesondere in der Haltestruktur axial angehoben oder gedreht werden. Die Behälter können gemäß weiteren Ausführungsformen auch in der Haltestruktur lyophilisiert werden, da ein direkter Bodenkontakt zu einem Kühlfinger des Gefriertrockenschranks möglich ist. Böden oder Köpfe der Behälter sind in einfacher Weise auf einer Höhe fixierbar, sodass sämtliche Böden in einer gemeinsam aufgespannten Ebene angeordnet werden können, was den unmittelbaren Kontakt zu flächigen Weiterverarbeitungstationen, insbesondere zu einem Kühlfinger bzw. einer Kühlhorde eines Gefriertrockenschranks ermöglicht. Ferner ist ein unmittelbarer Glas-Glas Kontakt von benachbarten Behältern über Trennstege oder dergleichen zuverlässig verhindert, was Abrieb und Verunreinigungen innerhalb der Weiterverarbeitungsanlage wirkungsvoll verhinder und so erheblich längeren Laufzeiten und Wartungsintervalle der Anlagen ermöglicht. Ferner können Kratzer oder die Erzeugung von Partikeln auf oder in den Behältern wirkungsvoll verhindert werden. Die erfindungsgemäße Haltestruktur ermöglicht dabei zweckmäßig die Entnahme der Behälter nach oben oder unten. Da die Position des Formschlusses zwischen Behälter und Haltestruktur einfach variiert werden kann, ist die erfindungsgemäße Haltestruktur sehr flexibel auch für Behälter mit unterschiedlichen Außenabmessungen einsetzbar. Insbesondere können die Behälter in der Haltestruktur in axialer Richtung einfach verschoben werden, sodass Behälter mit unterschiedlichen Höhen in derselben Haltestruktur gehalten werden können. Die axiale Verschieblichkeit der Behälter in der Haltestruktur ermöglicht auch einen einfachen Toleranzausgleich oder eine Weiterverarbeitung der Behälter, während diese in der Haltestruktur gehalten sind. So können diese beispielsweise von einer ersten Stellung axial in eine zweite Stellung verschoben werden und weiterhin in der Haltestruktur aufgenommen sein, wobei diese in der zweiten Stellung weiterverarbeitet werden können, beispielsweise deren Öffnungen mit einem Stopfen verschlossen werden oder auf den Stopfen ein äußerer Verschluss (beispielsweise Bördelkappe oder Krampe), häufig aus Aluminiumblech, aufgebracht werden. Gemäß weiteren Ausführungsformen können die Behälter, während diese in der Haltestruktur und in dem Transport- und Verpackungsbehälter aufgenommen sind, weiter verarbeitet werden. Geht dabei trotz aller Vorsichtsmaßnahmen dennoch ein Behälter zu Bruch, so führt dies nicht zu einer Verunreinigung der Anlage, da der Bruch aber auch etwaige Wirkstubstanzen oder -lösungen in dem Transport- und Verpackungsbehälter verbleiben.

Die Haltestruktur weist Haltemittel auf, wie nachfolgend ausführlicher dargelegt, die in einer regelmäßigen Anordnung angeordnet sind, insbesondere in einer Matrixanordnung entlang von Reihen und senkrecht dazu verlaufenden Spalten oder in einer Anordnung von regelmäßig zueinander versetzten Reihen von Öffnungen oder Aufnahmen. Die Anordnung der Haltemittel kann zur Erzielung einer möglichst hohen Packungsdichte der Behälter hinsichtlich ihrer Position optimiert werden.

Gemäß einer weiteren Ausführungsform ist zumindest ein überwiegender Teil der Böden oder unteren Enden der Behälter von einer Unterseite der Haltestruktur her frei zugänglich. Bevorzugt sind diese Flaschenböden von keinen Haltestrukturen oder dergleichen abgedeckt. Gemäß weiteren Ausführungsformen kann jedoch ein Haltesteg oder dergleichen am unteren Ende der Öffnungen bzw. Aufnahmen der Haltestruktur vorgesehen sein, um die Böden oder untere Ende der Behälter abzustützen. Somit kann eine Verstelleinrichtung, beispielsweise ein Verstellfinger, von der Unterseite der Haltestruktur her auf die Böden oder untere Enden der Behälter einwirken, um diese, während diese in der Haltestruktur gehalten sind, axial zu verstellen oder auch zu verdrehen. Beispielsweise können die Behälter so in der Haltestruktur geeignet angehoben und in der angehobenen Stellung und während diese in der Haltestruktur gehalten sind weiter verarbeitet werden, beispielsweise mit einem äußeren Verschluss (Bördelkappe oder Krampe) versehen werden. Für die Verstellung der Position der Behälter in der Haltestruktur kann gemäß weiteren Ausführungsformen die Öffnungsweite der Haltemittel, insbesondere die Öffnungsweite von Öffnungen oder Aufnahmen, verstellt werden, wie nachfolgend ausgeführt, sodass der Formschluss bei einer größeren Öffnungsweite aufgehoben oder wesentlich reduziert ist und bei einer geringeren Öffnungsweite zum zuverlässigen Halten der Behälter weiterhin besteht.

Ein weiterer Gesichtspunkt der vorliegenden Erfindung betrifft ferner einen Transport- und Verpackungsbehälter mit zumindest einer Haltestruktur, wie vorstehend ausgeführt und nachfolgend weiter im Detail offenbart.

Ein weiterer Gesichtspunkt der vorliegenden Erfindung betrifft ferner einen Transport- und Verpackungsbehälter mit Maßnahmen für einen Plagiatsschutz, insbesondere für eine Identifikation und/oder Nachverfolgung, wie nachfolgend ausgeführt.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a - 1c: einen Transport- und Verpackungsbehälter gemäß einer ersten Ausführungsform der vorliegenden Erfindung;
- Fig. 1d: eine Variante der Haltestruktur gemäß der Fig. 1a, die einer weiteren Erhöhung der Packungsdichte der Behälter dient;
- Fig. 1e - 1h: weitere Varianten für die formschlüssige Fixierung von Behältern bei einer Haltestruktur gemäß der Fig. 1a;
- Fig. 2a - 2c: einen Transport- und Verpackungsbehälter gemäß einer weiteren Ausführungsform der vorliegenden Erfindung;
- Fig. 3a - 3b: eine Haltestruktur gemäß einer weiteren Ausführungsform der vorliegenden Erfindung;
- Fig. 4a - 4c: eine Haltestruktur gemäß einer weiteren Ausführungsform der vorliegenden Erfindung;
- Fig. 5a - 5d: eine Haltestruktur gemäß einer weiteren Ausführungsform der vorliegenden Erfindung;
- Fig. 6: einen Transport- und Verpackungsbehälter gemäß einer weiteren Ausführungsform der vorliegenden Erfindung mit einer Schutz- oder Verpackungsfolie; und
- Fig. 7a - 7b: Maßnahmen zur Identifikation und/oder Nachverfolgung bei einem Transport- und Verpackungsbehälter gemäß der vorliegenden Erfindung.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur (im Stand der Technik häufig auch als "Nest" bezeichnet) sowie ein Transport- und Verpackungsbehälter (im Stand der Technik häufig auch als "Tub" bezeichnet), der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für kosmetische, medizinische oder pharmazeutische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder ein regelmäßigen Reihen, die relativ zueinander versetzt angeordnet sind.

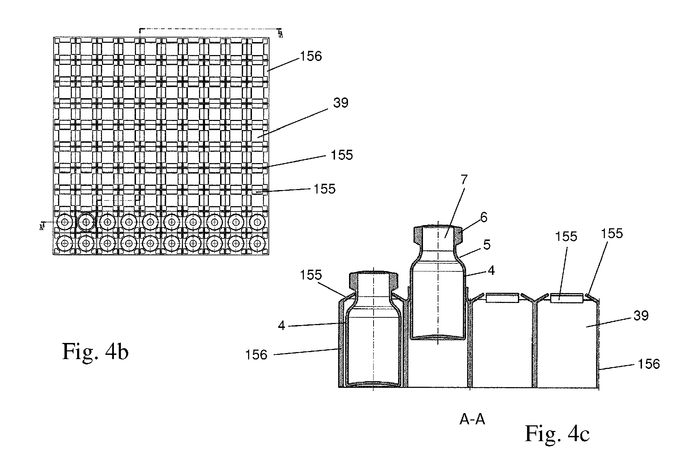

Ein Beispiel für derartige Medikamentenbehälter in Gestalt von Fläschchen (englisch: vial) ist in der Fig. 4c schematisch in einem Längsschnitt dargestellt und weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Glasbehälter erfindungsgemäß mittels eines Formschlusses an einer Halterungsstruktur fixiert. Dieser Formschluss wird im Bereich des verengten Halsabschnitts 5 realisiert. Insbesondere werden die Behälter formschlüssig im Bereich des verengten Halsabschnitts 5 abgestützt. Die Formschlusselemente, wie nachfolgend beschrieben, sind bevorzugt aus einem ausreichend flexiblen oder elastischen Kunststoff ausgebildet, sodass auch Glasbehälter mit großen Fertigungstoleranzen hinsichtlich ihrer axialen Länge im Übergangsbereich zwischen dem oberen Rand 6 und dem verengten Halsabschnitt 5 formschlüssig hintergriffen oder abgestützt werden können.

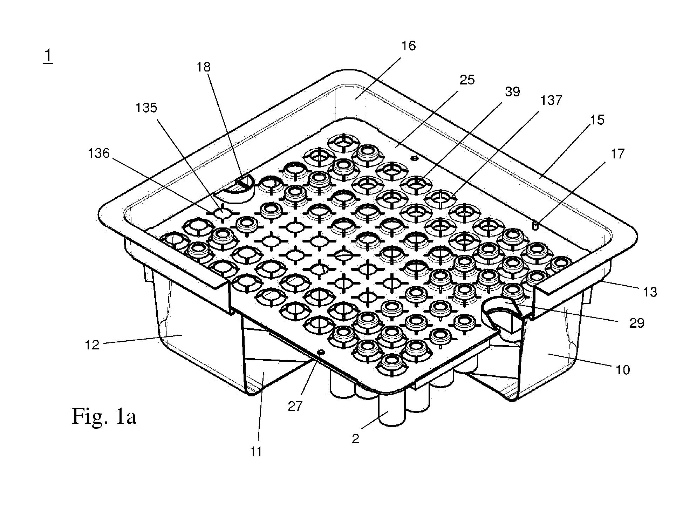

Zum gleichzeitigen Halten einer Mehrzahl von Behältern wird gemäß einer ersten Ausführungsform der vorliegenden Erfindung, wie in den Fig. 1a bis 1c dargestellt, eine flächige Transportplatte 25 bereitgestellt, die aus einem Kunststoff ausgebildet ist, beispielsweise ausgestanzt oder spritzgegossen ist, und die eine Mehrzahl von Öffnungen 39 zur Aufnahme der Glasfläschchen 2 aufweist. Diese Öffnungen sind in einer regelmäßigen Anordnung angeordnet, bei dem dargestellten Ausführungsbeispiel in einer Matrix-Anordnung aus Reihen und sich rechtwinklig dazu erstreckenden Spalten und unter gleichen Abständen zueinander. Alternativ können beispielsweise auch benachbarte Reihen von Behältern relativ zueinander regelmäßig versetzt sein.

Die Öffnungen sind in ringförmigen Formschlusselementen 137 ausgebildet, die nachfolgend noch ausführlicher beschrieben werden und die als Klappen wirken, die entweder in die Öffnungen 39 eingesteckt sind, insbesondere in deren Umfangsrand eingerastet oder eingeclipst sind, oder die einstückig mit dem flächigen Träger 25 ausgebildet sind, beispielsweise durch 1K- oder 2K-Kunststoff-Spritzgussverfahren. Die Zwischenräume zwischen den Formschlusselementen 137 können vollständig geschlossen ausgebildet sein oder als Öffnung ausgebildet sein. Die Behälter 2 können von oben oder von unten her in die Öffnungen 39 der Formschlusselemente 137 eingeführt werden. Dadurch können eine Mehrzahl von Behälter 2 im Bereich ihrer verengten Halsabschnitte 5 formschlüssig fixiert werden.

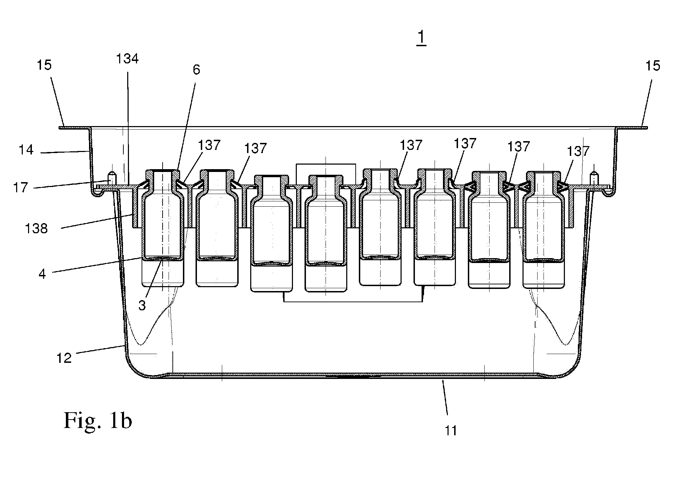

Dies ist in den schematischen Längsschnitten gemäß der Fig. 1b genauer dargestellt, wobei anzumerken ist, dass in der Fig. 1b summarisch mehrere verschiedene Ausführungsvarianten für Formschlusselemente 137 in einer vergleichenden Darstellung dargestellt sind. Im fixierten Zustand stützen die als Klappen wirkenden Formschlusselemente 137 die Behälter unmittelbar unterhalb des oberen Rands 6 und im Bereich des verengten Halsabschnitts 5. Wie in der Fig. 1b dargestellt, wird üblicherweise jedenfalls bei der großen Mehrzahl der fixierten Behälter der Abstand zwischen dem stufenartigen Übergangsbereich zwischen dem oberen Rand 6 und dem verengten Halsabschnitt 5 und der Oberseite der Formschlusselemente 137 verschwindend sein. Aufgrund einer gewissen Elastizität der Formschlusselemente 137 können jedoch in einem gewissen Umfang Fertigungstoleranzen der Behälter in axialer Richtung und auch Fertigungstoleranzen in radialer Richtung kompensiert werden und somit auch Behälter mit unterschiedlichen Durchmessern im Bereich des verengten Halsabschnitts 5 von ein- und derselben Haltestruktur 25 formschlüssig fixiert werden.

Eine Haltestruktur 25, wie in der Fig. 1a abgebildet, kann grundsätzlich in der Art eines Teppichs aufgerollt werden.

Zum Transport und zur Verpackung einer Haltestruktur im Sinne der vorliegenden Anmeldung mit den darin aufgenommenen Behältern dient ein Transport- und Verpackungsbehälter 10, wie schematisch in der Fig. 1a dargestellt (im Stand der Technik häufig auch als "Tub" bezeichnet). Gemäß der Fig. 1a ist der Behälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist. Die Ecken 16 des Behälters 10 sind zweckmäßig abgerundet ausgebildet. Die obere Seitenwand 14 kann unter einem geringen Neigungswinkel zur Senkrechten auf den Boden 11 geneigt ausgebildet sein, um das Einführen der Haltestruktur 25 zu erleichtern. Ein derartiger Behälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Behälter 10 aufgenommenen Haltestruktur 25 und der von dieser gehaltenen Behälter 2 zu ermöglichen.

Zur Aufnahme der Haltestruktur 25 in dem Behälter 10 kann diese von einem Halterahmen 26 umgeben sein, der einen geschlossen ausgebildeten Randsteg aufweist, oder jedenfalls entlang des Umfangsrands durchgehend ausgebildet sein. Zur zuverlässigen Positionierung der Haltestruktur 25 in dem Behälter 10 weisen die Haltestruktur 25 und der Behälter 10 miteinander zusammenwirkende Positionierungsgebilde auf, die insbesondere formschlüssig zusammenwirken. So können an geeigneter Stelle, insbesondere auf der Stufe 13 oder auf Abstützflächen 18 des Behälters 10 Positionierungsgebilde in Form von Vorsprüngen oder Aussparungen bzw. Vertiefungen ausgebildet sein, die mit korrespondierend ausgebildeten Aussparungen bzw. Vertiefungen oder Vorsprüngen der Haltestruktur formschlüssig zusammenwirken, um die Haltestruktur 25 präzise in dem Transportbehälter 10 zu positionieren. Zu diesem Zweck sind gemäß der Fig. 1a auf der Stufe 13 mehrere zapfenartige Vorsprünge 17 ausgebildet, die in korrespondierend ausgebildete Zentrieröffnungen 27 im Halterahmen 26 zusammenwirken. Gemäß der Fig. 1a ist die Stufe 13 des Behälters 10 als umlaufende, ebene Abstützfläche ausgebildet, auf welcher der Halterahmen 26 unmittelbar aufliegt. Gemäß weiteren Ausführungsformen können an den Seitenwänden 12 des Behälters 10 auch weitere Abstützflächen 18 oder Abstützelemente ausgebildet sein, insbesondere in Form von Vorsprüngen, wie nachfolgend ausgeführt. Auf diese Weise kann die Haltestruktur 25 präzise in dem Behälter 10 positioniert werden und die Mehrzahl von Behältern 2 auf diese Weise in einer regelmäßigen Anordnung und an präzise definierten Positionen in einem Behälter 10 mit standardisierten Abmessungen angeordnet und gehalten werden. Insbesondere kann auf diese Weise gewährleistet werden, dass sämtliche Böden oder untere Enden der Behälter 2 in einer gemeinsam aufgespannten Ebene parallel zum Boden 11 oder zum oberen Rand 15 des Behälters 10 angeordnet sind.

Wenngleich in der Fig. 1a der Boden 11 des Behälters 10 als geschlossen und einstückig mit der Seitenwand 12 ausgebildet dargestellt ist, kann das untere Ende des Behälters 10 auch in der Art des oberen Endes geöffnet ausgebildet sein, insbesondere mit einem flanschartigen unteren Rand in der Art des oberen Rands 15 versehen sein, so dass die Böden der Behälter 2 von der Unterseite des Behälters 10 her frei zugänglich sind, beispielsweise für Verarbeitungsschritte in einem Steriltunnel oder in einem Gefriertrockenschrank, wie nachfolgend näher erläutert.

Wie in der Fig. 1a dargestellt, sind in der regelmässigen Anordnung gemäß der Fig. 1a die Mehrzahl von Behälter 2 entlang von zwei zueinander orthogonalen Richtungen in einer Ebene unter vorbestimmten konstanten Abständen zueinander verteilt angeordnet. Grundsätzlich sind auch weitere regelmäßige Anordnungen denkbar, beispielsweise können zueinander benachbarte Reihen bzw. Spalten von Behältern 2 auch um eine vorbestimmte Länge versetzt zueinander angeordnet sein, und zwar in einer wiederkehrenden Anordnung mit vorbestimmter Periodizität. Somit können automatisierte Fertigungsanlagen die Behälter 2 bei Übergabe an eine Bearbeitungsstation an präzise vorgebbaren Positionen erwarten, was den Automatisierungsaufwand erheblich verringert. Wie nachfolgend näher erläutert, können erfindungsgemäß die Behälter 2 auch innerhalb der Haltestruktur 25 oder des Behälters 10 gemeinsam weiterverarbeitet werden, insbesondere auch in einem Steriltunnel oder einem Gefriertrockenschrank.

Damit die Haltestruktur 25 leicht in den Behälter 10 eingesetzt und aus diesem entnommen werden kann, sind an zwei Längsseiten des Halterahmens 26 Zugriffsöffnungen 29 ausgebildet, über welche Greifarme oder dergleichen die Haltestruktur 25 greifen können. Wie in der Fig. 1a erkennbar, sind die Zugriffsöffnungen 29 um eine Reihe versetzt zueinander angeordnet, was eine eineindeutige Positionierung der Haltestruktur 25 in dem Behälter 10 weiter erleichtert.

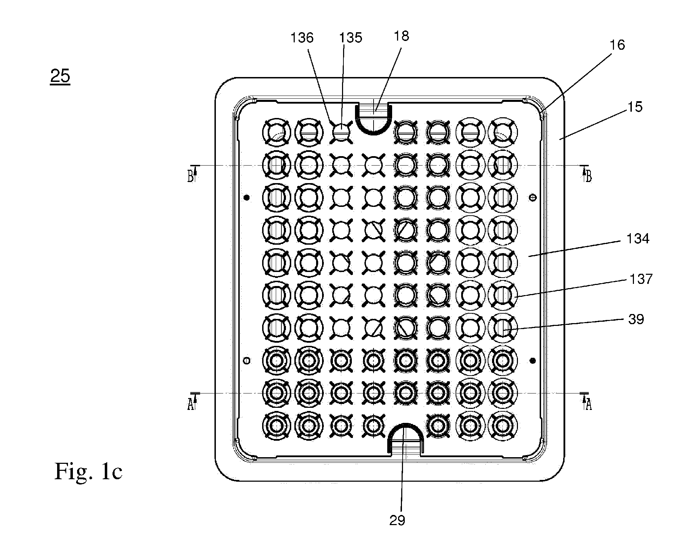

Die Fig. 1b zeigt einen schematischen Längsschnitt des Transport- und Verpackungsbehälters gemäß der Fig. 1a. Die Fig. 1c zeigt in einer Draufsicht und einer schematischen Perspektivansicht die Haltestruktur 25 gemäß der Fig. 1a.

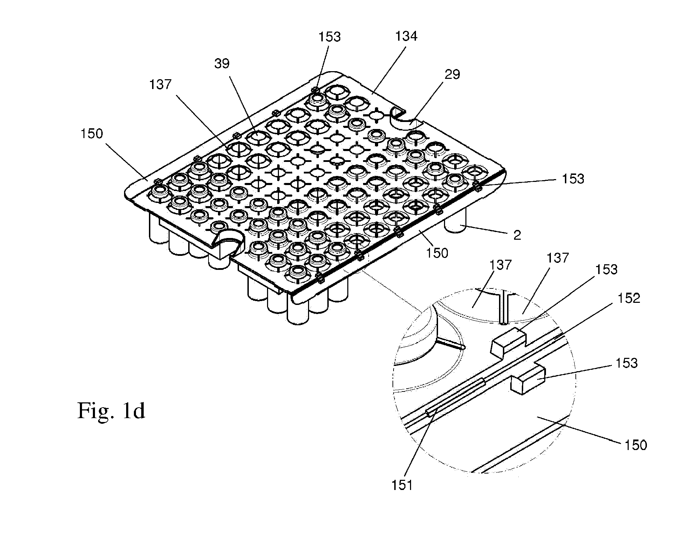

Die Fig. 1d zeigt eine weitere Variante der Haltestruktur gemäß der Fig. 1c, wobei Ränder 150 des plattenförmigen Trägers 134 wegeklappt werden können, um die Grundfläche des Trägers 134 weiter zu reduzieren, beispielsweise dann, wenn dieser mit den Behältern an eine beengte Weiterverarbeitungsstation übergeben werden soll, beispielsweise an einen Gefriertrockenschrank mit begrenzter Grundfläche. Zu diesem Zweck sind die Ränder 150 über Scharniere 151 mit dem Träger 134 verbunden. Auf der Oberseite des Trägers 134 und der Ränder 150 sind an einander entsprechenden Positionen blockförmige Anschläge 153 vorgesehen, die im gegenseitigen Anschlag eine koplanare Ausrichtung der Ränder 150 und des Trägers 134 festlegen. Gemäß einer weiteren Ausführungsform (nicht dargestellt) können die Ränder 150 auch von dem Träger 134 abgenommen werden. Die Ränder 150 können selbstverständlich entlang allen vier Längsseiten des Trägers 134 vorgesehen sein.

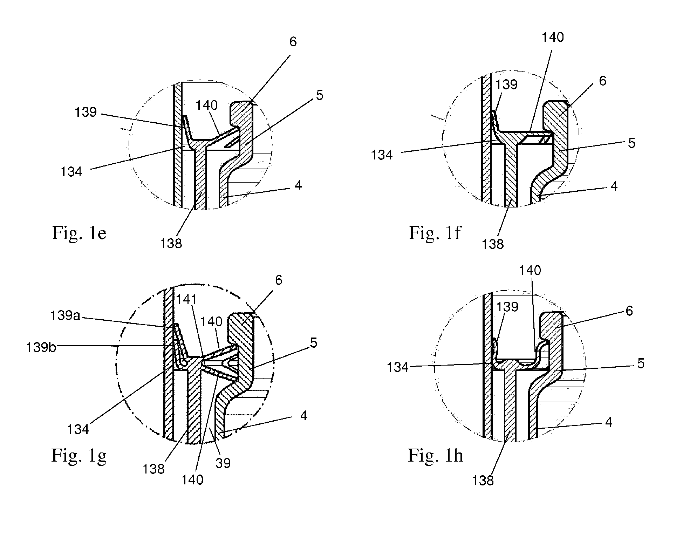

Die Figuren 1e bis 1h stellen weitere bevorzugte Varianten für die formschlüssige Fixierung der Behälter dar. Gemäß der Fig. 1e sind an dem Träger 134 als Klappen ausgebildete Formschlusselemente 140mit einem ringförmigen vorderen Ende vorgesehen, die den Halsabschnitt 5 formschlüssig umgreift und den oberen Rand 6 abstützt. Die Klappen 140 sind über den dargestellten Verbindungsbereich (ohne Bezugszeichen) mit dem Träger verbunden, wobei der Verbindungsbereich eine geringere Materialstärke aufweist, als die Klappe 140 und der Träger, sodass die Klappen 140 an dem Träger elastisch und schwenkbeglich gelagert sind. Gemäß der Fig. 1e sind an den unteren Enden der Klappen 140 Dichtlippen 139 vorgesehen. Beim Einführen des Behälters von oben oder unten her in die von den Klappen 140 ausgebildete Öffnung werden die Klappen 140 elastische zurückgeklappt und geben so die Öffnung in dem Träger frei. Durch Stärke, Material und Auslegung des Verbindungsbereichs zwischen Klappe 140 und Träger kann die ausgeübte Haltekraft sowie die zum Einführen und Entnehmen eines Behälters notwendige Kraft einfach vorgegeben werden. Mittels der schwenkbeweglich an dem Träger gelagerten Zunge 140 können auch Toleranzen von Behältern aufgrund von unterschiedlichen Außendurchmessern im Bereich des Halsabschnitts 5 einfach ausgeglichen werden. Grundsätzlich können dadurch auch Behälter unterschiedlichen Typs, beispielsweise mit unterschiedlichen Durchmessern im Bereich des Halsabschnitts 5, von derselben Haltestruktur gehalten werden.

Die Fig. 1f zeigt eine weitere Variante, bei der sich die Klappen 140 im Wesentlichen horizontal radial einwärts erstrecken, wenn der Behälter an dessen Halsabschnitt 5 gehalten ist. Zwischen dem vorderen Ende der Klappe 140 und dem Außenumfangsrand des Halsabschnitts 5 der Behälter kann ein gewisses radiales Spiel bestehen. Beim Einführen des Behälters von oben oder unten her in die von den Klappen 140 ausgebildete Öffnung werden die Klappen 140 elastisch zurückgeklappt, um die Öffnung in dem Träger freizugeben. Durch Stärke, Material und Auslegung des Verbindungsbereichs zwischen Klappe 140 und Träger kann die ausgeübte Haltekraft sowie die zum Einführen und Entnehmen eines Behälters notwendige Kraft einfach vorgegeben werden.

Gemäß der Fig. 1g weist das Formschlusselement zwei sich geneigt radial einwärts erstreckende Zungen 140 auf, die gemeinsamt an dem Träger schwenkbeweglich gelagert sind, wobei die oberen Zungen 140 den oberen Rand 6 des Behälters abstützen, während die unteren Zunge 140 im Übergangsbereich zwischen dem verengten Halsabschnitt 5 und der zylindrischen Seitenwand 4 der Behälteraußenwand anliegen. Stärke, Material und Auslegung des Verbindungsbereichs zwischen den oberen bzw. unteren Zungen 140 und Träger können identisch oder unterschiedlich sein. Mittels der oberen Zunge 140 kann die ausgeübte Haltekraft geeignet vorgegeben werden. Mittels der unteren Zunge kann die zum Einführen und Herausnahmen des Behälters notwendige Kraft geeignet vorgegeben werden. Die beiden Zunge n140 bilden insgesamt ein V-förmiges elastische Haltemittel, das den Halsabschnitt 5 formschlüssig umgreift und den oberen Rand 6 abstützt. Diese sind über einen Verbindungssteg 141 miteinander verbunden und über den Verbindungssteg 141 auch mit zwei seitlichen Dichtlippen 139a und 139b verbunden. Dadurch kann eine größere Stabilität bei der Fixierung der Behälter erzielt werden.

Gemäß der Fig. 1h ist die Klappe 140 S-förmig gebogen und somit in sich vorgespannt, was gleichfalls eine größere Stabilität bei der Fixierung der Behälter ermöglicht. Das Einführen und Entnehmen der Behälter kann hier bei geeigneter Wahl von Stärke, Material und Auslegung des Verbindungsbereichs zwischen Klappe 140 und Träger sanfter erfolgen. Insbesondere ist die Gefahr eines Verhakens der Behälters an der Klappen 140 hier noch weiter reduziert.

Wie den Figuren 1a bis 1h ohne Weiteres entnommen werden kann, können die Behälter von oben oder von unten in die Öffnungen 39 der Formschlusselemente eingeführt werden, um von diesen formschlüssig fixiert zu werden.

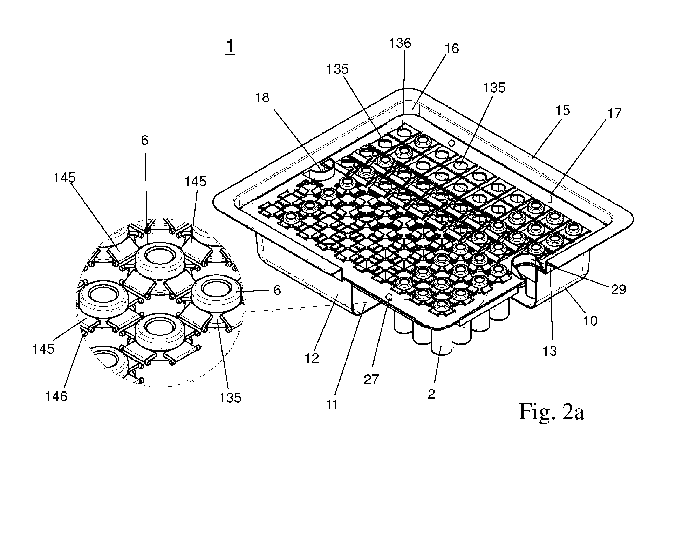

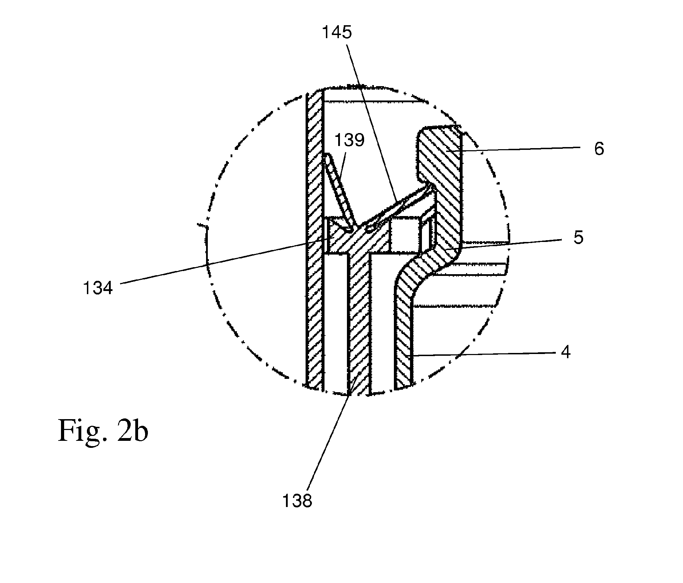

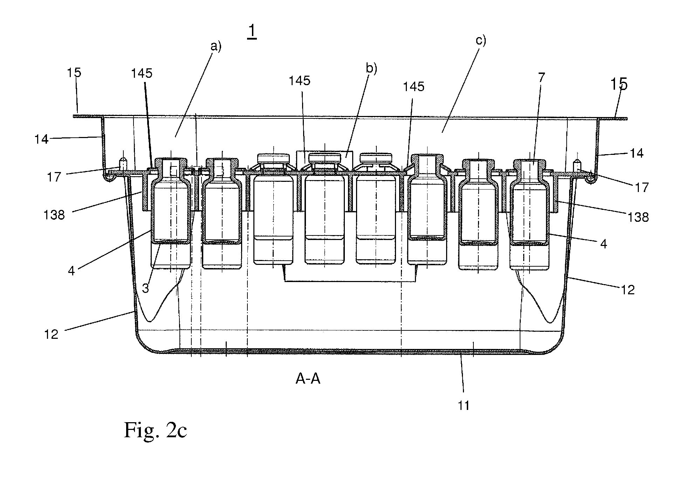

Die Figuren 2a und 2b zeigen eine weitere Ausführungsform, bei der Fixierung der Behälter mit Hilfe von schwenkbaren Haltezungen oder Klappen 145 realisiert ist, die schwenkbeweglich an dem Träger 134 gelagert sind. Die Haltezungen 145 weisen an ihrem unteren Ende jeweils einen seitlich abstehenden Zapfen 146 auf, der in eine korrespondierend ausgebildete Aufnahme eingeclipst oder eingedrückt ist. Die Haltezungen oder Klappen 145 können somit zwischen einer ersten Stellung, in welcher die Öffnungen 135 des Trägers freigegeben sind und die Behälter ungehindert eingeführt werden können, und einer zweiten Stellung geschwenkt werden, in welcher die Behälter formschlüssig fixiert sind. Die zum Verschwenken der Klappen bzw. Haltezungen 145 erforderliche Kraft kann insbesondere durch die Lagerung der Zapfen 146 in den hierzu vorgesehenen Aufnahmen geeignet vorgegeben werden.

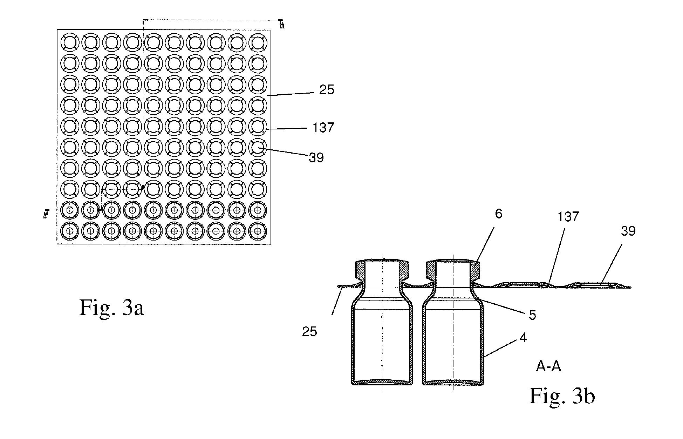

Die Figuren 3a und 3b zeigen eine weitere Variante des Trägers gemäß der Fig. 1c in einer schematischen Draufsicht und in einem Längsschnitt entlang A-A gemäß der Fig. 3a. Bei diesem Ausführungsbeispiel ist die Unterseite des Trägers 25 flach ausgebildet, während die Formschlusselemente 137 von der Oberseite des Trägers 25 abragen. Der Träger 25 nach dieser Ausführungsform kann einstückig mit einem Einkomponenten (1K)- oder Zweikomponenten (2K)-Spritzgussverfahren aus einem geeigneten Kunststoff hergestellt werden.

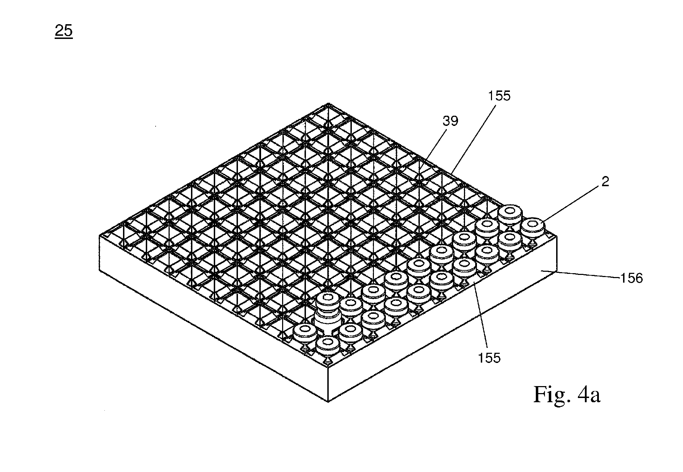

Die Figuren 4a bis 4c zeigen eine weitere Ausführungsform für einen Träger, der eine Mehrzahl von länglichen Aufnahmen 39 mit quadratischem Querschnitt aufweist, die von einer

Mehrzahl von unter gleichmässigen Abständen beabstandet zueinander angeordneten und sich rechtwinklig kreuzenden Querstegen 156 ausgebildet werden. Am oberen Rand der Querstege 156 sind jeweils rechteckförmige Klappen 155 angeordnet, die entweder einstückig mit den Querstegen ausgebildet sind oder verschwenkbar an deren oberem Rand gelagert sind. Gemäß der Fig. 4c, einem schematischen Längsschnitt entlang der Linie A-A gemäß der Fig. 4b, sind die Halsabschnitte 5 der Behälter formschlüssig von jeweils vier Klappen 155 umgriffen und die oberen Ränder 6 der Behälter auf diesen abgestützt. Die Behälter können nach oben hin aus den Aufnahmen 39 entnommen werden, wobei dabei die Klappen 155 weggeschwenkt werden.

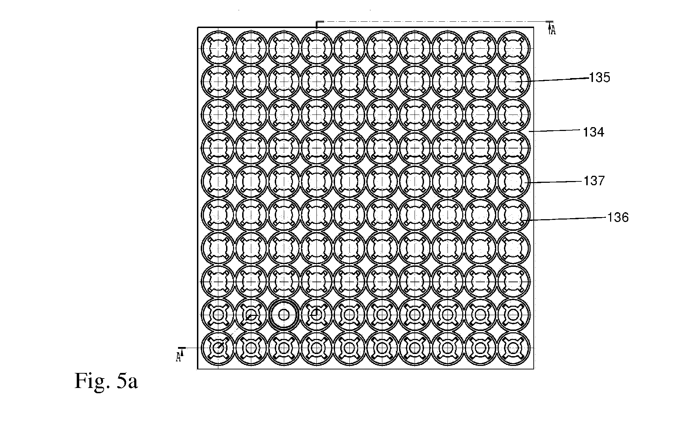

Die Figuren 5a bis 5d zeigen eine weitere Ausführungsform einer Haltestruktur bzw. eines Trägers 134 (im Stand der Technik auch als "Nest" bezeichnet). Auf der Oberseite des Trägers 134 sind in einer regelmäßigen Anordnung eine Mehrzahl von ringförmigen Aufnahmen ausgebildet, die von ringförmigen Seitenwänden 163 ausgebildet werden, deren Innendurchmesser auf den Außendurchmesser der darin aufzunehmenden Behälter abgestimmt sein kann. Wenn die Behälter in diesen Aufnahmen aufgenommen sein, sind die Böden der Behälter unmittelbar auf der Oberseite des Trägers 134 abgestützt, wobei die Seitenwände 163 zuverlässig eine Berührung von benachbarten Behältern verhindern. Die Aufnahmen können dabei die unteren Enden der Behälter 2 klemmen.

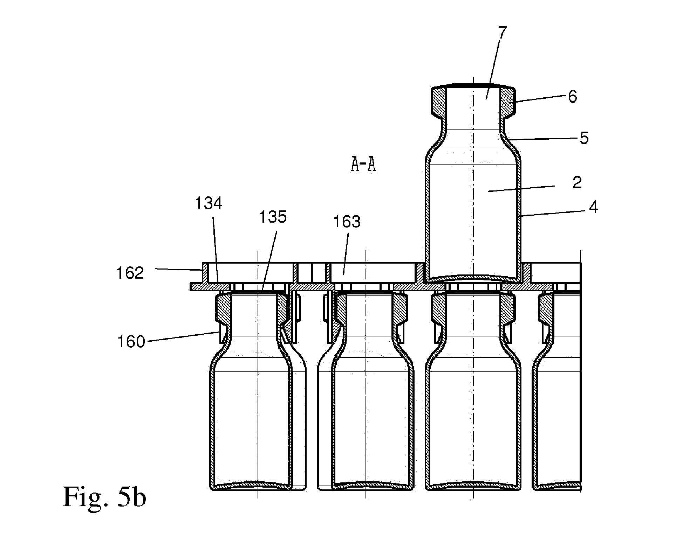

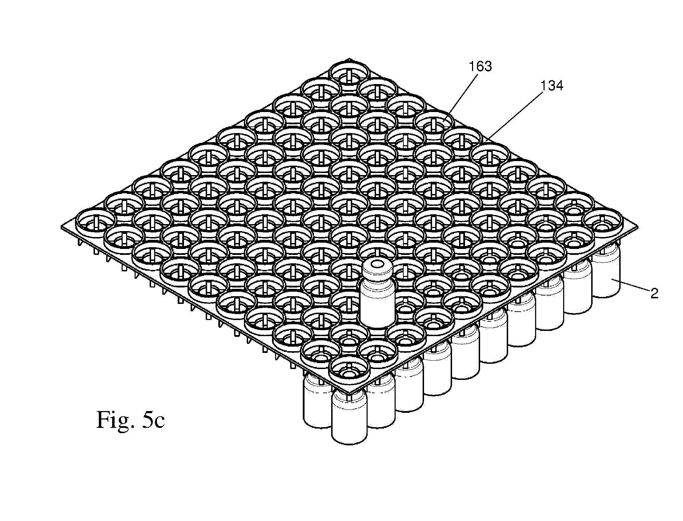

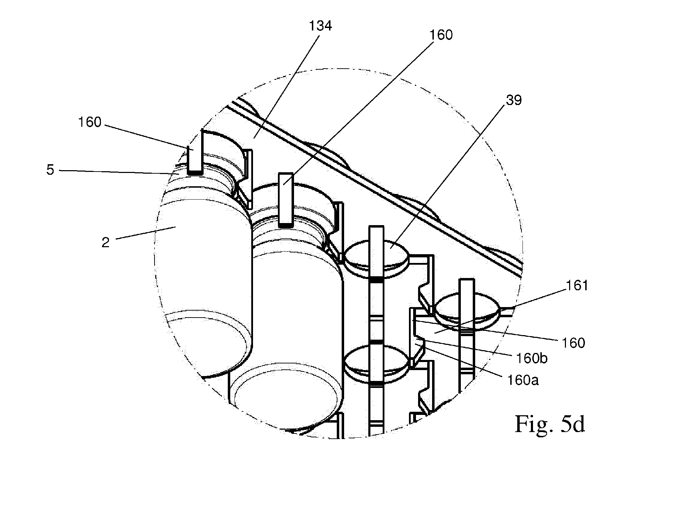

Gemäß der vergrößerten Darstellung nach der Fig. 5d, einem stark vergrößerten Ausschnitt aus der Fig. 5c, ragen von der Unterseite des Trägers hakenförmige Rasthaken 160 ab, die um die Öffnungen 135 des Trägers 134 herum angeordnet sind. Die Behälter 2 können von unterhalb des Trägers 134 her in die Rasthaken 160 eingeschoben werden, bis der ober Rand 6 der Behälter von den Rasthaken 160 formschlüssig hintergriffen ist. In dieser Stellung sind die Behälter zuverlässig gelagert, wobei sämtliche Böden der Behälter 2 auf demselben Höhenniveau angeordnet sind und eine Berührung benachbarter Behälter verhinder ist. Die Rasthaken 160 sind jedoch ausreichend elastisch, sodass die Behälter 2 auch daraus wieder unter elastischer Verformung entnommen werden können. Wie man der Fig. 5b und 5c entnehmen kann, können mehrere Lagen aus solchen Trägern 134 mit den daran fixierten Behältern übereinander gestapelt werden.

Wie man den Figuren 5b und 5d entnehmen kann, weisen die Rasthaken 160 eine untere Einführschräge 160a auf. Beim Einführen der Behälter 2 von der Unterseite des Trägers her gelangt schließlich der obere Rand 6 der Behälter 2 in Anlage mit der unteren Einführschräge 160a. Beim weiteren Annähern der Behälter 2 an den Träger gleitet der obere Rand 6 der Behälter entlang der unteren Einführschräge 160a, sodass die Rasthaken elastisch radial auswärts geschwenkt werden und so auseinander gespreizt werden. Schließlich gleitet der untere Rand des oberen Rands 6 der Behälter 2 (vgl. Fig. 5b) über die engste Stelle der Rasthaken 160 und in der Folge schnellen der Rasthaken 160 zurück in ihre Ausgangslage, in welcher die Behälter 2 im Bereich des Halsabschnitts 5, insbesondere untermittelbar unterhalb des oberen Rands 6, von den Rasthaken 160 geklemmt gehalten werden können. Gemäß einer weiteren Ausführungsform kann die Unterseite des oberen Rands 6 der Behälter auch auf der schräg abwärts geneigten Auflagefläche 160b der Rasthaken lose aufliegen, d.h. mit einem gewissen radialen Spiel. Wie man der Fig. 5d entnehmen kann, erleichtert die Neigung der schräg abwärts geneigten Auflagefläche 160b ein Entfernen der Behälter 2 von dem Träger. Denn beim Abziehen der Behälter 2 nach unten gleitet die Unterseite des oberen Rands der Behälter entlang der schräg abwärts geneigten Auflagefläche 160b, anschließend über die engste Stelle der Rasthaken. Beim weiteren Abziehen der Rasthaken 160 schnellen diese schließlich elastisch in Ihre Ausgangslage zurück.

Während bei den vorherigen Ausführungsbeispielen dargelegt wurde, dass die Behälter aufrecht und mit ihrem offenen Ende zum oberen Ende des Transport- und Verpackungsbehälters hin angeordnet sind, können die Behälter grundsätzlich auch gewendet, d.h. mit ihrem offenen Ende hin zum Boden des Transport- und Verpackungsbehälters zeigend, angeordnet sein.

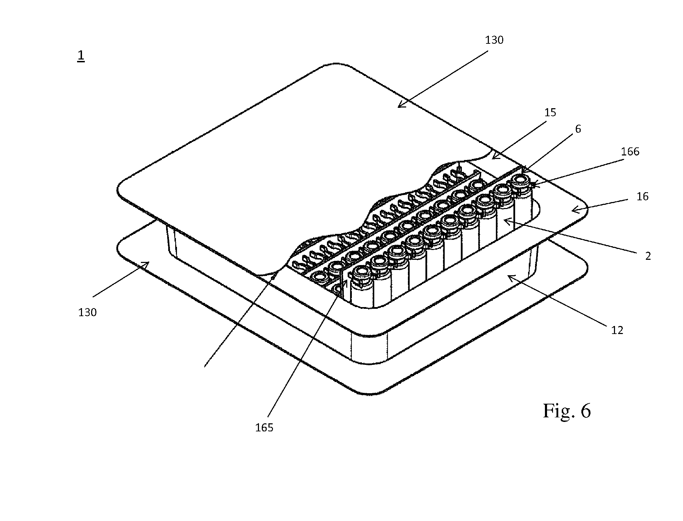

Die Fig. 6 zeigt eine Verpackungseinheit, die von einem beidseitig offenen Transportbehälter 12 und einer darin aufgenommen Haltestruktur gemäß 165 gemäß einer hier nicht weiter erläuterten Ausführungsform ausgebildet ist und auf der Ober- und Unterseite mittels einer auf den Rand 15 des Transportbehälters 12 aufgeklebten Schutz- oder Schutz- oder Verpackungsfolie 130 verschlossen ist. Bei der Schutzfolie 130 kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 durch die Folie 130 hindurch ermöglicht. Wie dem Fachmann ohne weitere ersichtlich ist, kann der Transportbehälter 12 auch nur an einem Ende geöffnet ausgebildet sein, etwa in der Art des Transport- und Verpackungsbehälters 10, wie dieser schematisch in der Fig. 1a dargestellt ist. Bei sämtlichen Ausführungsformen der Erfindung können dem Kunststoff der Haltestruktur (des Trägers) und/oder des Transport- und Verpackungsbehälters antimikrobiell wirkende Pulver beigemischt sein. Antimikrobielle wirkende Pulver, beispielsweise mit Ag, Zn, Cu, Ce, Te oder I Atomen oder Ionen, sind aufgrund ihrer bioziden, bakterioziden, und fungiziden Wirkung als Zuschlagstoff oder Füllstoff für die unterschiedlichsten Zwecke geeignet. Die biozide Wirkung tritt beispielsweise gegenüber Bazillen, Pilzen, Viren, Hefen, Algen und anderen Kleinlebewesen ein. Als biozid wirkende Komponenten können dem Kunststoff der Haltestruktur (des Trägers) und/oder des Transport- und Verpackungsbehälters insbesondere auch Glaspulver zugegeben werden, die Ag₂O, CuO, Cu₂O, TeO₂, ZnO, CeO₂ und I enthalten. Für die Anwendung in Kunststoffen bleiben die mechanischen und optischen Eigenschaften weitgehend unverändert. Die Einbringung der Pulver kann hierbei entweder bereits als Nanopulver beispielsweise den Kunststoffpulvermischungen beim Spritzguss oder in Lacken bei Tiefziehprozessen mit eingebracht werden. Eine antimikrobielle Wirkung kann auch erzielt werden, wenn der Kunststoff mit einer antimikrobiellen Beschichtung versehen ist, welche die vorgenannten Pulver enthalten.

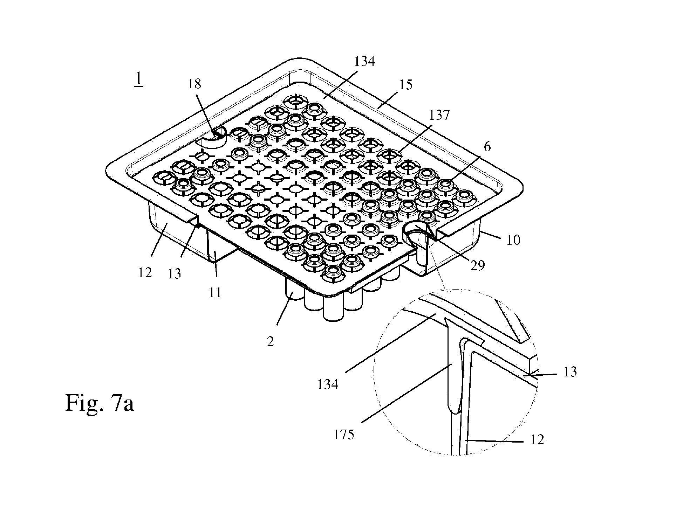

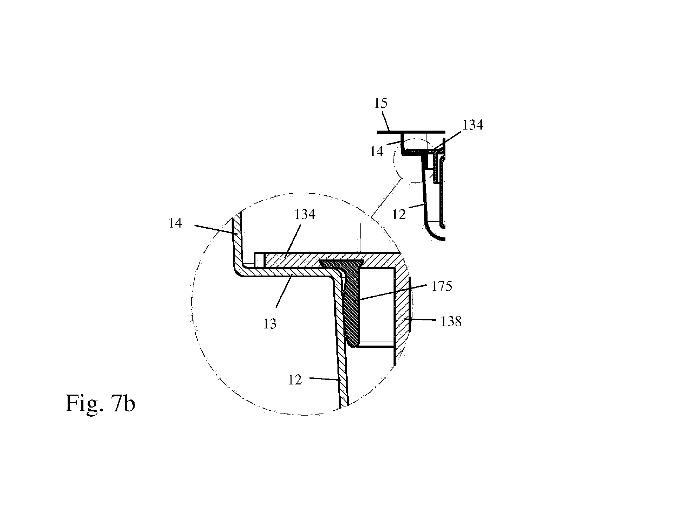

Die Fig. 7a und 7b zeigen eine weitere Ausführungsvariante für eine Verpackungseinheit 1 mit einer in dem Transport- und Verpackungsbehälter 10 aufgenommenen Halteplatte 134, die nur einer beispielhaften Erläuterung dienen soll und in welcher eine Mehrzahl von Behältern 2 mittels Ringelementen 137 formschlüssig fixiert sind. Die Verpackungseinheit 1 weist Vorkehrungen zur Identifikation und/oder Nachverfolgung wie folgt auf: Wie in dem vergrößerten Einsatz der Fig. 11a dargestellt, ist in dem Bereich der Zugriffsaussparung 29 zwischen die Halteplatte 134 und die Seitenwand 12 und/oder die Stufe 13 des Behälters 10 ein elektronisch drahtlos (wireless) auslesbarer RFID-Chip oder RuBee-Chip 175 angeordnet (ein RuBee Chip funkt auf Frequenzen, die Metall- und Wasser durchdringen können), der durch die Seitenwände der Verpackungseinheit 1 hindurch berührungslos ausgelesen werden kann und auf Abfrage Information bezüglich der Identität, wichtige Produkteigenschaften (Hersteller, Inhalt, Herstellungsdatum, Verfallsdatum, ...) etc. ausgibt. Der Chip 175 kann in die Verpackungseinheit 1 an geeigneter Stelle eingeklebt sein, auch an anderer Stelle als in der Figur dargestellt. Der Chip 175 kann so angeordnet sein, dass im Falle eines Öffnens der Verpackungseinheit 1 oder eines Herausnehmens der Halteplatte 134 aus der Verpackungseinheit 1 heraus der Chip 175 zerstört wird, beispielsweise zerrissen oder funktionsunfähig wird. Aufgrund der fehlenden Antwort des Chips 175 auf eine Funkabfrage hin steht somit eine Information zur Verfügung, die anzeigt, dass die Verpackungseinheit seit der vorherigen Verpackung in irgendeiner Weise manipuliert worden sein muss. Denn der Chip 175 reagiert auf die Funkabfrage nicht. Dies kann beispielsweise zum Nachweis der Echtheit und Unversehrtheit der Verpackungseinheit und der darin aufgenommenen Behälter dienen.

Gemäß einer weiter bevorzugten Ausführungsform ist der RuBee- oder RFID Chip 175 in Kombination mit weiteren Sensoren integriert, die wichtige Parameter des Transport- und Verpackungsbehälters 1 zeitabhängig überwachen können, welche wichtige Qualitäts- oder Echtheitseigenschaften der in dem Transport- und Verpackungsbehälter 1 aufbewahrten Behälter betreffen. Diese Qualitäts- oder Echtheitseigenschaften können periodisch aufgezeichnet und in einem dem Chip 175 oder Sensor zugeordneten Speicher abgelegt werden. Zur elektrischen Stromversorgung dieser elektronischen Bauelemente kann in dem Transport- und Verpackungsbehälter 1 eine netzunabhängige Spannungsversorgung vorgesehen sein, insbesondere eine Batterie mit geringen Abmessungen oder auch induktiv über eine kleine Leiterschleife. Als Sensoren sind gemäß der vorliegenden Anmeldung insbesondere angedacht:
- ein Feuchtigkeitssensor mit oder ohne Speicher (Data-Logging), der die in dem Transport- und Verpackungsbehälter vorherrschende Luftfeuchtigkeit periodisch misst und bei Bedarf aufzeichnet;
- ein Gassensor mit oder ohne Speicher (Data-Logging), der die Konzentration von in dem Transport- und Verpackungsbehälter vorhandenen Gasen, wie beispielsweise O₂, Ozon, CO₂ oder von Entkeimungsgase, wie z.B. Ethylenoxyd, Formaldehyd, misst und bei Bedarf aufzeichnet;
- ein Temperatursensor mit oder ohne Speicher (Data-Logging), der die in dem Transport- und Verpackungsbehälter vorherrschende Temperatur periodisch misst und bei Bedarf aufzeichnet;
- ein UV Sensor mit oder ohne Speicher (Data-Logging), der in den Transport- und Verpackungsbehälter eindringende UV-Strahlung periodisch misst und bei Bedarf aufzeichnet;
- ein Gammastrahlungs-, Elektronenstrahlen- oder Röntgenstrahlensensor mit oder ohne Speicher (Data-Logging), der in den Transport- und Verpackungsbehälter eindringende Strahlung periodisch misst und bei Bedarf aufzeichnet;

Ferner können in dem Transport- und Verpackungsbehälter weitere Vorkehrungen getroffen werden, die einen Plagiatschutz und einen zuverlässigen Nachweis der Authentizität und Echtheit der in dem Transport- und Verpackungsbehälter aufbewahrten Fläschchen betreffen. Hierzu kann insbesondere ein Lumineszenzplagiatschutz vorgesehen sein, und zwar in Form von an geeignetem Ort in oder an dem Transport- und Verpackungsbehälter aufgebrachten Leuchtstoffen, die herkömmlich auch als "Phosphore" bezeichnet werden. Die Leuchstoffe, die für das menschliche Auge bevorzugt unsichtbar sind, sind jedoch anhand von unterschiedlichen spektralen Emissionsspektren charakteristisch unterscheidbar. Die Leuchstoffe bestehen bevorzugt aus anorganischen Lumineszenzmaterialien (z.B. Mikrokristalle, Nanokristalle und/oder Quantumdots), die bei bestimmten Wellenlängen fluoreszieren und/oder phosphoreszieren. Die verschiedenen Arten der Lumineszenz werden nach der Dauer des Leuchtens nach Ende der Erregung eingeteilt in Fluoreszenz (< 1 msec) und Phosphoreszenz (≥ 1 msec).

Die Emissionswellenlängen können eine geringe spektrale Breite aufweisen (z.B. InBO₃:Eu oder Tb) und/oder aber auch breit emittieren (Ce:YAG). Die spektrale Lage hängt von der Zusammensetzung und/oder auch der Konzentration der Leuchtstoffe ab. Die Herstellung erfolgt durch Mischung der Leuchtstoffe mit Kunststoffgranulat, -Pulver, Lösungsmitteln und oder Lacken vor der Weiterverarbeitung (Spritzguss, Tiefziehen, ...). Auch organische Lumineszenzmaterialien können Anwendung finden.

Man ist also in der Lage verschiedene Verpackungschargen herzustellen, die charakteristische Emissionsspektren aufweisen, deren Emissionslinien von der eingesetzten Konzentration und der Kombination von Fluoreszenzmaterialien (z.B. Oxyde, , Oxynitride, Nitride, Sulfide, Fluoide, ...) sich in der Wellenlänge und den Intensitätsverhältnissen unterscheiden. Die Lumineszenzmaterialien können z.B. zusammengesetzt sein aus unterschiedlichen Eu dotierten Materialien wie CaS:Eu, Sr₂Si₅N₈:Eu, SrS:Eu, Ba₂Si₅N₈:Eu, Sr₂SiO₄:Eu, SrSi₂N₂O₂:Eu, SrGa₂S₄:Eu, SrAl₂O₄:Eu, Ba₂SiO₄:Eu, Sr₄Al1₄O₂₅:Eu, SrSiAl₂O₃N:Eu, BaMgAl₁₀O₁₇:Eu, Sr₂P₂O₇:Eu, SrB₄O₇:Eu, Y₂O₃:Eu, YAG:Eu, Ce:YAG:Eu, (Y,Gd)BO₃:Eu, (Y,Gd)₂O₃:Eu. Lumineszenzmaterialien können co-dotiert oder auch mit anderen Seltenen Erden (Scandium, Yttrium, Lanthan, Cer, Praseodym, Neodym, Promethium, Samarium, Gadolinium, Terbium, Dysprosium, Holmium, Erbium, Thulium, Ytterbium und Lutetium) dotiert werden (z.B. LaPO₄:Ce,Tb, LaMgAl₁₁O₁₉:Ce,Tb, (Y,Gd,Tb,Lu)AG:Ce, Lu_{3-x-z}AₓAl_{5-y-z}Sc_{y}O₁₂:Mn_{z}Ca_{z}, Lu₂SiO₅:Ce, Gd₂SiO₅:Ce, Lu_{1-x-y-a-b}YₓGd_{y})₃ (Al_{1-z}Ga)₅O₁₂:CeₐPr_{b} ). Günstige Lumineszenzmaterialien für VUV Anregung sind LaPO₄:Pr, YPO₄:Pr, (Ca,Mg)SO₄:Pb, LuBO₃:Pr, YBO₃:Pr, Y₂SiO₅:Pr, SrSiO₃:Pb, LaPO₄:Ce, YPO₄:Ce, LaMgAl₁₁O₁₉:Ce. Bei Anregung mit Rontgenstrahlen können beispielhaft die folgenden Lumineszenzmaterialien verwendet werden: InBO₃:Tb+InBO₃:Eu, ZnS:Ag, Y₂O₂S:Tb, Y₂SiO₅:Tb, Y₃(Al,Ga)₅O₁₂:Ce,

(Zn,Cd)S:Cu,Cl+(Zn,Cd)S:Ag,Cl, Y₃(Al,Ga)₅O₁₂:Tb, Zn₂SiO₄:Mn, Zn₈BeSi₅O₁₉ :Mn, CaWO₄:W, Y₂O₂S:Eu+Fe₂O₃, (Zn,Mg)F₂:Mn, Y₃Al₅O₁₂:Tb.

Rein rechnerisch lassen sich damit mehrere 100 Milliarden Fluoreszenzvarianten herstellen. Eine Identifizierung ist mit einfachen Handspektrometern möglich.

Eine Erweiterung des Plagiatschutzes kann erreicht werden, wenn einige Emissionsspektren genutzt werden um andere Lumineszenzmaterialien anzuregen. D.h. die Anregung erfolgt beispielsweise mittels einer UVC-Bestrahlung (z.B. Wellenlänge=254 nm). Der Leuchstoff emittiert bei UVA (z.B. Wellenlänge=450 nm), wodurch andere Lumineszenzmaterialien angeregt werden. Weiterhin kann man anhand der zeitlich unterschiedlichen Emissionen (Decay time) zusätzlich einen zeitlich charakteristisches Fingerabdruck erhalten. Auch durch die Verwendung von Isotopen erhält man z.T. spektrale Isotopieverschiebungen, die optisch nachgewiesen werden können. Dadurch sind mehrere Trillionen Kombinationen denkbar. Damit lassen sich insbesondere unsichtbare Fluoreszenz-Markierungen im und auf dem Transport- oder Verpackungsbehälter mit Anregungsspektren im Rontgenspektrum (10pm- 1 nm), extremen UV (1nm-100nm), VUV (100nm-200nm), UVC (200nm-280nm), UVB (280nm-320nm), UVA (320nm-400nm), und/oder im blauen Spektralgebiet (400nm-480nm) realisieren. Die Anregung kann mittels verschiedener Lichtquellen, wie beispielsweise Rontgenröhren, Laser, LEDs, Halogenlampe und/oder Kaltkathodenlampen (cold cathode lamps) kontinuierlich oder gepulst und/oder kombiniert realisiert werden. Die Emissionsspektren der Fluoreszenzmarkierungen können im UV, sichtbaren Spektralgebiet (VIS) und/oder im infraroten Spektralgebiet (NIR und/oder MIR) liegen. Die Detektion kann mit einem handelsüblichen Spektrometer und oder auch zeitaufgelöst beispielsweise mit Hilfe eines Boxcarverstärkers erfolgen, um mittels Detektion in einem Zeitfenster den zeitlichen Abfall der Emission zeitaufgelöst zu messen und/oder auch zu zwei verschiedenen Zeiten im Zeitabstand und nachzuweisen. Der Zeitabstand kann beispielweise von einigen ns bis zu einigen ms betragen und sollte länger sein, als die Abschaltzeit der Lichtquelle. Es können auch verschiedene Pigmente (z.B. Nanokristalle) kombiniert werden, die zeitlich, örtlich und/oder spektral unterschiedlich fluoreszieren. Weiterhin können die Lumineszenzmarkierungen auch in Form von 1D Barcodes (z.B. EAN, UPC, IAN, JAN) und/oder 2D Barcodes (z.B. QR, Data Matrix, Maxi, Punkt) und/oder als Composite Code angeordnet werden. Die Barcodes können entweder direkt auf die Verpackung mit z.B. Siebdruckverfahren, Tintenstrahldruckverfahren oder speziellen Sprühverfahren mit Schablone aufgebracht werden oder indirekt über Etiketten

(Kunststoff oder Papier) aufgeklebt werden. Auch das Aufbringen von mehreren additiven oder strukturierten Lumineszenzschichten ist möglich.

Die von den formschlüssig wirkenden Haltemitteln jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Gemäß weiteren Ausführungsformen kann die Haltekraft durch geeignete Auslegung der Haltemittel auch so bemessen sein, dass diese größer ist als übliche Kraft bei der Handhabung, Verarbeitung oder Behandlung der Behälter in einer Prozessanlage. Dadurch wird stets eine zuverlässige Halterung der Behälter gewährleistet. Gleichwohl können die Behälter gemäß bevorzugten weiteren Ausführungsformen der Erfindung gegen diese Haltekraft in den Öffnungen oder Aufnahmen verstellt werden, insbesondere axial vorgeschoben oder gedreht werden. Die hierfür erforderliche Kraft muss nur größer sein als die von den Haltemitteln ausgeübte Kraft.

Selbstverständlich kann die Haltestruktur (der Träger) im Sinne der vorliegenden Erfindung auch aus einem thermoplastischen, duroplastischen oder elastomeren Kunststoff ausgebildet sein, zumindest Abschnitte der Haltestruktur bzw. des Trägers mit einer reibreduzierenden Beschichtung versehen sind, um das Einführen und die Entnahme der Behälter zu erleichtern.

Gemäß weiteren Ausführungsform kann die Haltestruktur und/oder der Transportbehälter, oder Abschnitte davon, aus einem faserverstärkten Kunststoffe oder einem Kunststoff ausgebildet sein, dem zur Erhöhung seiner Wärmeleitfähigkeit Keramiken oder Metalle beigegeben beigegeben sind. Bekanntermaßen haben faserverstärkte Kunststoffe eine höhere Wärmeleitfähigkeit bis 0,9 W/(K m) bei Kohlenstofffasern. Werden den Kunststoffen Keramiken oder Metalle beigegeben, so wird die Wärmeleitfähigkeit weiter vergrößert. Es entstehen die sogenannten wärmeleitenden Kunststoffe. So wird eine Wärmeleitfähigkeit von 20 W/(K m) erreicht.

Gemäß einer weiteren Ausführungsformen werden somit erfindungsgemäß bereitgestellt:
Ausführungsform A Eine Haltestruktur zum gleichzeitigen Halten einer Mehrzahl von Behältern (2) für Substanzen für medizinische, pharmazeutische oder kosmetische Anwendungen, mit einem flächigen, rechteckförmigen Träger (25; 134), der eine Mehrzahl von Öffnungen oder Aufnahmen (32; 39; 87; 120), die in einer regelmäßigen Anordnung angeordnet sind, und diesen zugeordnete Haltemittel aufweist, um die Mehrzahl von Behältern (2) an dem Träger formschlüssig zu halten **dadurch gekennzeichnet, dass** die Haltemittel als Klappen (145; 155) ausgebildet sind, die um die Öffnungen (135) oder Aufnahmen (39) des Trägers (25) herum verteilt angeordnet sind, wobei die einer jeweiligen Öffnung oder Aufnahme zugeordneten Klappen (145) in einer ersten Stellung, in welcher die Öffnung oder Aufnahme vollständig freigegeben ist, weggeklappt sind und in einer zweiten Stellung in Richtung der Öffnung zurück geklappt sind, um gemeinsam einen oberen Rand (6) des in der jeweiligen Öffnung oder Aufnahme aufgenommenen Behälters abstützen.
Ausführungsform B Eine Haltestruktur nach Ausführungsform A, wobei die Klappen einstückig mit dem Träger ausgebildet sind.
Ausführungsform C Eine Haltestruktur nach Ausführungsform B, wobei die Klappen über einen Verbindungsbereich mit dem Träger verbunden sind, wobei der Verbindungsbereich eine geringere Materialstärke aufweist als der Träger und/oder die Klappen, sodass die Klappen schwenkbeweglich und elastisch an dem Träger gelagert sind.
Ausführungsform D Eine Haltestruktur nach Ausführungsform A, wobei die Klappen formschlüssig mit dem Träger verbunden sind.
Ausführungsform E Haltestruktur nach Ausführungsform D, wobei die Klappen seitlich abragende Zapfen aufweisen, die in korrespondierend ausgebildete Aufnahmen an dem Träger eingeclipst sind.
Ausführungsform F Eine Haltestruktur nach einer der Ausführungsformen A bis E, wobei die vorderen Enden der Klappen (145) eine kreissegmentförmige Aussparung aufweisen, deren Krümmungsradius auf den Außenradius der zu haltenden Behälter abgestimmt ist.
Ausführungsform G Eine Haltestruktur nach einer der Ausführungsformen A bis E, wobei die Klappen (145) rechteckförmig ausgebildet sind. Ausführungsform H Eine Haltestruktur nach einer der Ausführungsformen A bis G, wobei die Klappen (145) am oberen Rand der zugeordneten Aufnahmen (39) vorgesehen
   sind, wobei die Aufnahmen (39) einen rechteckförmigen oder quadratischen Querschnitt aufweisen, dessen Öffnungsweite auf den Außendurchmesser der zu haltenden Behälter abgestimmt ist.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Haltestruktur zum gleichzeitigen Halten einer Mehrzahl von Behältern (2) für Substanzen für medizinische, pharmazeutische oder kosmetische Anwendungen, wobei die Behälter (2) einen verengten Halsabschnitt (5) aufweisen, mit
einem flächigen, rechteckförmigen Träger (25; 134), der eine Mehrzahl von Öffnungen oder Aufnahmen (32; 39; 87; 120) aufweist, die in einer regelmäßigen Anordnung angeordnet sind und die ausgelegt sind, sodass die Behälter in die zugeordneten Öffnungen oder Aufnahmen einführbar sind, und
Haltemitteln (145; 155), die einer jeweiligen Öffnung oder Aufnahme zugeordnet sind und ausgelegt sind, um die Mehrzahl von Behältern (2) an dem Träger (134) an vorbestimmten Positionen und in der regelmäßigen zweidimensionalen Anordnung zu halten,
**dadurch gekennzeichnet, dass** die Haltemittel als Formschlusselemente (137; 140; 145; 155) ausgebildet sind, die als Klappen wirken, um die Behälter im Bereich ihrer verengten Halsabschnitte (5) an dem Träger formschlüssig zu fixieren.

2. Haltestruktur nach Anspruch 1, wobei Formschlusselemente (145; 155) um die Öffnungen (135) oder Aufnahmen (39) des Trägers (25) herum verteilt angeordnet sind, wobei die Formschlusselemente in einer ersten Stellung, in welcher die jeweilige Öffnung oder Aufnahme vollständig freigegeben ist, weggeklappt sind und in einer zweiten Stellung in Richtung der jeweiligen Öffnung oder Aufnahme zurück geklappt sind, um gemeinsam einen oberen Rand (6) des in der jeweiligen Öffnung oder Aufnahme aufgenommenen Behälters abstützen.

3. Haltestruktur nach Anspruch 1 oder 2, wobei die Formschlusselemente einstückig mit dem Träger ausgebildet sind, insbesondere mit Hilfe eines 1K- oder 2K-Kunststoff-Spritzgussverfahrens.

4. Haltestruktur nach einem der vorhergehenden Ansprüche, wobei zwischen den Formschlusselementen einer jeweiligen Öffnung oder Aufnahme als Öffnungen ausgebildete Zwischenräume vorgesehen sind.

5. Haltestruktur nach einem der vorhergehenden Ansprüche, wobei die Formschlusselemente (137) eine gewisse Elastizität aufweisen, sodass Fertigungstoleranzen der Behälter (2) in axialer Richtung oder in radialer Richtung kompensiert werden können und auch Behälter mit unterschiedlichen Durchmessern im Bereich des verengten Halsabschnitts (5) an dem Träger formschlüssig fixiert werden können.

6. Haltestruktur nach einem der vorhergehenden Ansprüche, wobei die vorderen Enden der Formschlusselemente (145) eine kreissegmentförmige Aussparung aufweisen, deren Krümmungsradius auf den Außenradius der zu haltenden Behälter abgestimmt ist.

7. Haltestruktur nach Anspruch 5 oder 6, wobei die Formschlusselemente über einen Verbindungsbereich mit dem Träger verbunden sind, wobei der Verbindungsbereich eine geringere Materialstärke aufweist als der Träger und/oder die Formschlusselemente, sodass die Formschlusselemente schwenkbeweglich und elastisch an dem Träger gelagert sind.

8. Haltestruktur nach einem der vorhergehenden Ansprüche, wobei zwischen den vorderen Enden der Formschlusselemente und dem Außenumfang des verengten Halsabschnitts des jeweils in den Öffnungen oder Aufnahmen gehaltenen Behälters (2) ein radiales Spiel besteht.

9. Haltestruktur nach einem der vorhergehenden Ansprüche, wobei die Formschlusselemente jeweils als V-förmige Haltemittel ausgebildet sind, die eine sich geneigt radial aufwärts erstreckende Zunge zum Abstützen des oberen Rands (6) der Behälter und eine sich geneigt radial abwärts erstreckende Zunge aufweisen, die in einem Übergangsbereich zwischen dem verengten Halsabschnitt (5) und einer zylindrischen Seitenwand (4) der Behälter anliegen, wobei die beiden Zungen über einen Verbindungssteg (141) miteinander verbunden sind.

10. Haltestruktur nach einem der vorhergehenden Ansprüche, wobei die Haltestruktur in der Art eines Teppichs aufgerollt werden kann.

11. Haltestruktur nach einem der vorhergehenden Ansprüche, wobei die Formschlusselemente schwenkbeweglich an dem Träger (134) gelagert sind, wobei die Formschlusselemente (145) seitlich abragende Zapfen (146) aufweisen, die in korrespondierend ausgebildete Aufnahmen an dem Träger (134) eingeclipst sind, wobei die Formschlusselemente bevorzugt rechteckförmig ausgebildet sind.

12. Haltestruktur nach einem der vorhergehenden Ansprüche, wobei die Formschlusselemente (145) am oberen Rand der zugeordneten Aufnahmen (39) vorgesehen sind, wobei die Aufnahmen (39) einen rechteckförmigen oder quadratischen Querschnitt aufweisen, dessen Öffnungsweite auf den Außendurchmesser der zu haltenden Behälter (2) abgestimmt ist.

13. Haltestruktur nach einem der vorhergehenden Ansprüche, wobei Randabschnitte (68) des Trägers (134) abnehmbar oder wegschwenkbar sind, um eine Grundfläche der Haltestruktur insgesamt zu verringern.

14. Haltestruktur nach einem der vorhergehenden Ansprüche, wobei die Haltemittel so ausgelegt sind, dass zumindest ein überwiegender Teil der Böden (3) oder unteren Enden der Behälter von der Unterseite des Trägers her frei zugänglich sind und dass die Behälter (2) so an dem Träger (25) gehalten sind, dass sämtliche Böden oder untere Enden der Behälter unter demselben Abstand zur Oberseite des Trägers (25) angeordnet oder anordenbar sind, um gemeinsam eine Ebene aufzuspannen.

15. Transport- oder Verpackungsbehälter für eine Mehrzahl von Behältern (2) für Substanzen für medizinische oder pharmazeutische Anwendungen, umfassend einen kastenförmigen Behälter (10), **gekennzeichnet durch** eine in dem kastenförmigen Behälter (10) aufgenommene Haltestruktur (25) nach einem der vorhergehenden Ansprüche zum gleichzeitigen Halten der Mehrzahl von Behältern.
